# EUROPEAN PATENT APPLICATION

(11) **EP 4 389 158 A1**
(43) Date of publication of application: **26.06.2024**
(21) Application number: 22214539.3
(22) Date of filing: 19.12.2022
(51) Int. Cl.: A61L 2/07, A61L 2/24

(54) **A STEAM STERILIZER SYSTEM**

(71) Applicant: Getinge Sterilization AB, 305 05 Getinge (SE)
(72) Inventor: STORBERG, Bengt, 311 70 Falkenberg (SE)
(74) Representative: Zacco Sweden AB

(57) **Abstract**

A steam sterilizer system, comprising a sterilizer chamber configured to receive articles to be sterilized, a steam generator configured to turn liquid water into steam to be supplied to the sterilizer chamber, the steam generator comprising a heat exchanger, and a steam recycling arrangement configured to receive residual steam that is present in the sterilizer chamber after a steam treatment stage, wherein the steam recycling arrangement is configured to pass the residual steam, from the sterilizer chamber, as a heat transfer medium through said heat exchanger so as to transfer heat from the residual steam to the liquid water in the steam generator, wherein the steam recycling arrangement comprises a compressor configured to receive the residual steam from the sterilizer chamber and compress the received residual steam to a higher pressure before the steam recycling arrangement passes the compressed residual steam to the heat exchanger.

## Description

### TECHNICAL FIELD

The present disclosure relates to a steam sterilizer system. Steam sterilizer systems can be used for processing used medical goods in a hospital central sterile services department (CSSD), for example.

### BACKGROUND ART

Inactivation of microorganisms by means of a steam sterilization process involves the presence of steam (moist heat) and high temperatures over a certain time period. During such a steam sterilization process, the material to be sterilized must be in contact with the steam and its condensate for said time period. The steam sterilization process typically comprises a number of different treatment stages, and may include several evacuations. In current systems such evacuations purge the residual steam to the sewage system. Such evacuations waste a large amount of energy since the residual steam still contains much energy. However, recovering the residual steam to avoid wasting energy is difficult since the residual steam, even though it is hot, is not hot enough for additional steam sterilizations. A further consideration of the evacuation of residual steam is the considerable amount of water that is required to cool the residual steam in order to protect the vacuum system and to make release into the sewage system possible. It would also be desirable to reduce such water consumption.

### SUMMARY OF THE INVENTION

An object of the present disclosure is to present a steam sterilizer system which at least partly alleviates the efficiency drawbacks of the prior art. This and other objects, which will become apparent in the following, are accomplished by a steam sterilizer system as defined in claim 1. Some non-limiting exemplary embodiments are presented in the dependent claims.

The applicant has determined that residual steam should be passed from a sterilizer chamber through a compressor so as to compress the received residual steam to a higher pressure, thereby increasing the temperature of the residual steam and enabling the energy of the residual steam to be recovered. This also reduces water consumption in connection with draining residual steam to the sewage system.

Thus, according to at least a first aspect of the present inventive concept, there is provided a steam sterilizer system, the steam sterilizer system comprising:
- a sterilizer chamber configured to receive articles, such as medical goods, to be sterilized in a sterilization process in the sterilizer chamber,
- a steam generator configured to receive liquid water and turn the liquid water into steam to be supplied to the sterilizer chamber, the steam generator comprising a heat exchanger, and
- a steam recycling arrangement configured to receive at least a part of the residual steam that is present in the sterilizer chamber after a steam treatment stage of the sterilization process, wherein the steam recycling arrangement is configured to pass the residual steam, from the sterilizer chamber, as a heat transfer medium through said heat exchanger so as to transfer heat from the residual steam to the liquid water in the steam generator,
wherein the steam recycling arrangement comprises a compressor configured to receive the residual steam from the sterilizer chamber and compress the received residual steam to a higher pressure before the steam recycling arrangement passes the compressed residual steam to the heat exchanger.

Compressing the residual steam to a higher pressure and thereby to a higher temperature requires less energy than the energy that would be required to achieve the same temperature by other traditional heating means. The heat energy of the compressed residual steam can then effectively be used as a heat transfer medium for heating fresh water in the steam generator. This has an additional benefit: Any contaminants, debris or other undesired particles which may have followed the residual steam out form the sterilizer chamber will not be recirculated into the sterilizer chamber since the residual steam will merely be used as a heat transfer medium to enable steam to be created from fresh water in the steam generator.

Furthermore, since the compressed residual steam will be used as a heat transfer medium in the heat exchanger it will effectively be cooled as heat is transferred to the fresh water in the steam generator. The compressed residual steam having passed through the heat exchanger will have a lower temperature and may form condensate that can be evacuated to the sewage system with less or no water cooling. Thus, the steam sterilizer system of the present disclosure can avoid wasting the considerable amounts of water that has traditionally been used for cooling residual steam evacuated from a sterilizer chamber before being release into the sewage system. Furthermore, instead of evacuating it to the sewage system, it may be conceivable to recover the condensate or any remaining residual steam that has passed through the heat exchanger.

Different sterilization processes may comprise different numbers and types of steam treatment stages. For instance, a sterilization process may start with a subatmospheric pre-treatment for air removal with vacuum followed by steam injection, which both may be repeated several times to create a diluted atmosphere which mainly comprises steam that is free from air. The first subatmospheric stage may be followed by a superatmospheric pre-treatment through steam injection to a higher pressure followed by evacuation down close to atmospheric pressure, which is done repeatedly to heat the load and further remove residual air and accumulated Non-Condensable Gases (NCG). A third stage of the pre-treatment may include controlled heating for steam conditioning up to sterilization temperature. The sterilization then takes place through holding constant temperature for a certain time. Sterilization may then be followed by post-treatment e.g. with vacuum drying. Some stages may be more suitable for residual steam recovery than others. Steam evacuated from subatmospheric pre-treatment may be more or less contaminated with air and is also colder/thinner than later in the process. Thus, a later stage may be more advantageous for residual steam recovery. Furthermore, at later stages, the pressure of the residual steam will be higher than in the early subatmospheric pre-treatment stage. Therefore, the later stages require a lower compression ratio than the early stage. The residual steam from the superatmospheric pre-treatment and from a portion of the post-treatment is relatively free from air or NCG and also quite hot and dense, which makes it particularly suitably for recovery in compression with a reasonable compression ratio. Nevertheless, it should be understood that the steam sterilizer system of this disclosure is not limited to recovering residual steam from late steam treatment stages of a sterilization process. The steam sterilizer system may also be operated to recover residual steam during evacuations earlier in pre-treatment. In some preferred embodiments, residual steam is recovered from steam sterilization stages conducted at superatmospheric (high pressure) and/or high temperature conditions.

According to at least one exemplary embodiment, the steam recycling arrangement comprises an intermediate steam storage to which the compressed residual steam is guided before the compressed residual steam is passed on to the heat exchanger, the pressure in the intermediate steam storage preferably being higher than the pressure in the steam generator, in particular wherein the intermediate steam storage comprises a pressure vessel. Having a higher pressure in the intermediate steam storage compared to the pressure in the steam generator improves the heat transfer. As a purely illustrative example, the pressure in the intermediate steam storage may, for instance, be 500-900 kPa, while the pressure in the steam generator may, for instance, be 360-410 kPa. It should also be understood that, in operation, the pressure in the intermediate steam storage is preferably higher than the pressure in the sterilizer chamber.

From the above, it can be understood that, in accordance with at least one exemplary embodiment, the compressor is located in a fluid path between the sterilizer chamber and the intermediate steam storage for compressing the residual steam from the sterilizer chamber before it enters the intermediate steam storage.

The intermediate steam storage may be provided with its own heating arrangement, such as one or more electric heaters, since not all the energy may be recovered from the residual steam. Furthermore, such a heating arrangement may be useful during start-up of the intermediate steam storage from a cold state before sufficient hot steam is available for recovery.

The intermediate steam storage may potentially receive superheated steam from the compressor, in which case it may be appropriate to reduce the temperature to avoid excessive heat. Superheated steam provides poor heat transfer in the heat exchanger of the steam generator. It may therefore be advantageous to control any superheat before it reaches the heat exchanger. Limiting the superheat before or during the compression stage may also protect the compressor from excessive temperatures. However, it is also conceivable to limit the superheat downstream of the compressor. Thus, it should be understood that there are be several conceivable strategies for controlling superheat. Some examples will be discussed in relation to the following exemplary embodiments.

According to at least one exemplary embodiment, the intermediate steam storage has an inlet at the bottom of the intermediate steam storage through which compressed residual steam is receivable, thereby enabling residual steam to rise through water contained in the intermediate steam storage. The presence of water in the intermediate steam storage is moderating the residual steam and will counteract any superheat, and can control the residual steam back to saturation. A steam saturation table may be used for controlling the temperature based on the pressure in the intermediate steam storage. Accordingly, the intermediate steam storage may suitably be provided with various sensing devices, such as a pressure sensor and/or a temperature sensor. The intermediate steam storage may suitably, in normal operation, typically contain both water and steam. The water will have a moderating effect on the residual steam, and can also be used for generating new steam (e.g. by electric heaters or another heating arrangement) when the system starts up. Also after start-up, the water may be used for adding new steam in order to compensate for losses.

According to at least one exemplary embodiment, the intermediate steam storage comprises a screen with a plurality of holes for spreading the transportation of steam bubbles through a water volume in the intermediate steam storage, wherein the steam bubbles rise to an upper steam volume above the surface of the water volume. By distributing the residual steam over a larger surface the superheat may be reduced or controlled.

Instead of, or in addition to, integrating the superheat control in the intermediate steam storage as exemplified above, the steam recycling arrangement may be provided with any other suitable cooling device. This is at least partly reflected in the following exemplary embodiments.

In a general sense, according to at least one exemplary embodiment, the steam recycling arrangement may comprise a cooling device configured to lower the temperature of superheated residual steam into saturated residual steam, in particular wherein the cooling device is along a fluid path between the sterilizer chamber and an intermediate steam storage.

According to at least one exemplary embodiment, the cooling device is configured to spray water into the compressor. This may be beneficial as the material of the compressor may be better protected from the superheated residual steam. The cooling device may for instance comprise a nozzle, wherein water may be spray-injected into the residual steam flow with the nozzle and with help from a feed water pump. The water may, for instance, be sprayed into the decreasing compression volume of the compressor during a stroke, which will cause evaporation and instant cooling directly when superheat occurs. This may be aligned to start in a certain spot along one revolution during the compression stroke, e.g. by reading a position sensor which may be provided on the compressor. The duration may then be controlled by a controller to reach saturation temperature without going into the wet area, e.g. using feedback from a temperature sensor and pressure sensor to calculate the saturation temperature. It is, however, also conceivable to spray water without aligning the injection with the compression stroke.

According to at least one exemplary embodiment, the cooling device may be in the form of an injector configured to spray water onto superheated residual steam upstream or downstream of the compressor. A position downstream of the compressor will not provide protection to the internal components of the compressor from high temperatures, as positions upstream of the compressor or directly into the compression stroke does, however, may be convenient from a assembling perspective. A position downstream of the compressor can be in a piping or even directly into the intermediate steam storage. According to at least one exemplary embodiment, the cooling device may be in the form of a heat exchanging device provided between the compressor and the intermediate steam storage.

According to at least one exemplary embodiment, the steam sterilizer system further comprises a control unit configured to determine that a steam treatment stage of the sterilization process has been completed and, based on such determination, control a valve located upstream of the compressor to allow residual steam to pass from the sterilizer chamber to the compressor. A technical benefit may include that the control unit may be programmed to open the valve at those stages of the sterilization process that are considered to allow the compressor to operate at a reasonable compression ratio for achieving the desired pressure and temperature for the received residual steam. The control unit may also be used for controlling the operation of other parts of the steam sterilizer system.

The control unit may include a microprocessor, microcontroller, programmable digital signal processor or another programmable device. The control unit may also, or instead, include an application specific integrated circuit, a programmable gate array or programmable array logic, a programmable logic device, or a digital signal processor. Where it includes a programmable device such as the microprocessor, microcontroller or programmable digital signal processor mentioned above, the processor may further include computer executable code that controls operation of the programmable device. The control unit is preferably configured for use with steam sterilizer systems, and provided with electronic instructions to perform and control any operational steps described herein. The control unit may include any electronics and electronic instructions for controlling the processes described herein.

According to at least one exemplary embodiment, the intermediate steam storage is a first intermediate steam storage, wherein the steam recycling arrangement comprises a second intermediate steam storage to which the compressed residual steam is guidable before the compressed residual steam is passed to the heat exchanger. The second intermediate steam storage may have a lower pressure than the first intermediate steam storage. For instance, the second intermediate steam storage may have a pressure in the range of 150-300 kPa. The second intermediate steam storage may be used for compression of steam held in saturated balance with water which in turn may be used for pre-heating of feed water in economizers, or its contents may be used for a second compression stage from the lower steam pressure in the second intermediate storage to the higher pressure in the first intermediate steam storage. Having two intermediate steam storages may conserve more energy as the lowest possible pressure for evacuation of the sterilizer chamber can be much lower since the compression ratio also becomes lower. The same compressor may suitably be used for different operating modes and flow may be directed by controlling different valves in piping between the sterilizer chamber, the first intermediate steam storage and the second intermediate steam storage.

According to at least one exemplary embodiment, the control unit is configured to operate the steam recycling arrangement in a first storage operating mode by controlling the compressed residual steam to be initially provided to the first intermediate steam storage until the pressure inside the sterilizer chamber has dropped below a predefined pressure level. When the pressure in the sterilizer chamber is high, it is advantageous to provide the compressed residual steam to the first intermediate steam storage, because the compression ratio will be relatively low. However, when the pressure in the sterilizer chamber drops, the compression ratio will after a while become too large for efficiently continuing in this first storage operating mode. Therefore, the control unit may switch the passing of compressed residual steam to the second intermediate steam storage.

Thus, according to at least one exemplary embodiment, the control unit is configured to operate the steam recycling arrangement in a second storage operating mode when the pressure inside the sterilizer chamber has dropped below said predefined pressure level, by controlling the compressed residual steam to be provided to the second intermediate steam storage instead of to the first intermediate steam storage. This is advantageous since the second intermediate steam storage provides a lower counter-pressure.

According to at least one exemplary embodiment, the control unit is configured to operate the steam recycling arrangement in an inter-storage operating mode by controlling the compressed residual steam stored in the second intermediate steam storage to be forwarded to the first intermediate steam storage via a compressor to further increase the pressure of the residual steam exiting the second intermediate steam storage. This may be the same compressor positioned in the path between the sterilizer chamber and the first intermediate steam storage, or it may be a separate compressor in a fluidic path between the first and second intermediate steam storages. This is advantageous as this inter-storage operating mode may increase pressure and energy in the first intermediate steam storage for subsequent heat transfer in the steam generator. It should be noted, however, that it is also conceivable to use energy from the second intermediate storage for direct heating.

According to at least one exemplary embodiment, the steam recycling arrangement comprises a plurality of controllable valves for controlling the flow of steam to and from the first and second intermediate steam storages, wherein the control unit is configured to control the controllable valves to switch between said different operating modes. By using a plurality of controllable valves, it is possible in some embodiments to only use one compressor for all three operating modes.

According to at least one exemplary embodiment, the sterilizer chamber is a first sterilizer chamber and the steam generator is a first steam generator, wherein the steam sterilizer system further comprises:
- a second sterilizer chamber configured to receive articles, such as medical goods, to be sterilized,
- a second steam generator configured to receive liquid water and turn the liquid water into steam to be supplied to the second sterilizer chamber, the second steam generator comprising a heat exchanger,
- wherein the steam recycling arrangement is configured to also guide the residual steam as a heat transfer medium through the heat exchanger of the second steam generator so as to transfer heat from the residual steam to the liquid water in the second steam generator. Thus, the steam sterilizer system is not limited to one sterilizer chamber, but can advantageously include one or more additional sterilizer chambers. The compressor may advantageously be used to provide compressed residual steam received from the first sterilizer chamber to the heat generator of either one of the first steam generator or the second steam generator. Similarly, in at least some exemplary embodiments, residual steam from the second steam sterilizer may be received by the compressor and then be forwarded to the heat exchanger of either one of the first or second steam generator. A common intermediate steam storage may suitably be provided from to which compressed residual steam is passed before being forwarded to one of steam generators. In other exemplary embodiments, there may be provided a respective intermediate steam storage.

According to at least one exemplary embodiment, the steam recycling arrangement further comprises a bypass line extending from a location downstream of the compressor but upstream of the steam generator, wherein the bypass line is selectively controllable to allow compressed residual steam to bypass the steam generator and be returned to the sterilizer chamber. Such exemplary embodiments may or may not include the provision of one or more intermediate steam storages (such as those discussed above). In embodiments including an intermediate steam storage, the bypass line may extend either upstream of or downstream of the intermediate steam storage. In any case, an advantage of having a bypass line is that there is no conversion losses (as compared to the heat transfer at the heat exchanger in the steam generator).

The use of a bypass line may be considered as also representing a second general aspect of the present disclosure.

Thus, according to at least a second aspect of the general inventive concept, there is provided a steam sterilizer system, comprising:
- a sterilizer chamber configured to receive articles, such as medical goods, to be sterilized in a sterilization process in the sterilizer chamber,
- a steam generator configured to receive liquid water and turn the liquid water into steam to be supplied to the sterilizer chamber, and
- a steam recycling arrangement configured to receive at least a part of the residual steam that is present in the sterilizer chamber after a steam treatment stage of the sterilization process, wherein the steam recycling arrangement comprises a compressor configured to receive the residual steam from the sterilizer chamber and compress the received residual steam to a higher pressure,
wherein the steam recycling arrangement further comprises a bypass line extending from a location downstream of the compressor but upstream of the steam generator, wherein the bypass line is selectively controllable to allow compressed residual steam to bypass the steam generator and be returned to the sterilizer chamber.

Thus, this second aspect, unlike the first aspect, does not require the compressed residual steam to be used as a heat transfer medium in a heat exchanger of the steam generator. Therefore, there will be no conversion losses, because no heat transfer is conducted in this second aspect, rather the compressed residual steam as such may be reused as the sterilizing medium in the sterilizer chamber. To reduce the risk of unwanted matter re-entering the sterilizer chamber, the steam sterilizer system may suitably recover residual steam from a late stage of the sterilization process.

Generally, all terms used in the claims are to be interpreted according to their ordinary meaning in the technical field, unless explicitly defined otherwise herein. All references to "a/an/the element, arrangement apparatus, component, means, step, etc." are to be interpreted openly as referring to at least one instance of the element, arrangement apparatus, component, means, step, etc., unless explicitly stated otherwise. The steps of any method disclosed herein do not have to be performed in the exact order disclosed, unless explicitly stated. Further features of, and advantages with, the present inventive concept will become apparent when studying the appended claims and the following description. The skilled person realizes that different features of the present inventive concept may be combined to create embodiments other than those described in the following, without departing from the scope of the present inventive concept.

### BRIEF DESCRIPTION OF THE DRAWINGS

Fig. 1 is a schematic view of a steam sterilizer system according to at least one exemplary embodiment of the present disclosure having one intermediate steam storage.
Fig. 2 is a schematic view of a steam sterilizer system according to at least another exemplary embodiment of the present disclosure having two intermediate steam storages.
Fig. 3 illustrates an example of a steam sterilizer which could be used with the disclosed system.
Fig. 4 illustrates an example of a steam generator which could be used with the disclosed system.

### DETAILED DESCRIPTION

The present disclosure will now be described more fully hereinafter with reference to the accompanying drawings, in which certain aspects of the system are shown. The system may, however, be embodied in many different forms and should not be construed as limited to the embodiments and aspects set forth herein; rather, the embodiments are provided by way of example so that this disclosure will be thorough and complete, and will fully convey the scope of the system to those skilled in the art. Accordingly, it is to be understood that the present disclosure is not limited to the embodiments described herein and illustrated in the drawings; rather, the skilled person will recognize that many changes and modifications may be made within the scope of the appended claims. Like reference numerals refer to like elements throughout the description.

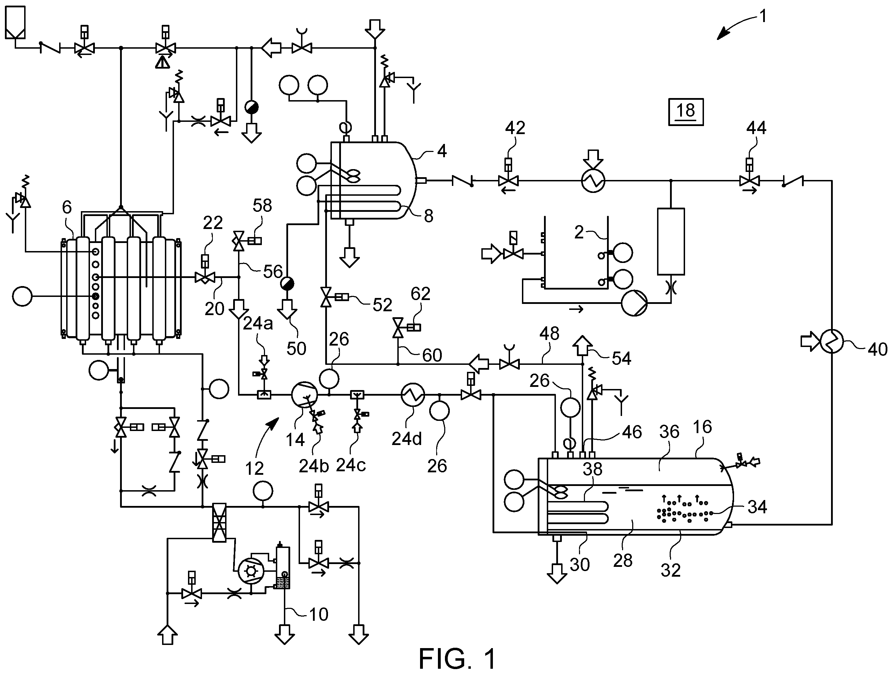

Fig. 1 is a schematic view of a steam sterilizer system 1 according to at least one exemplary embodiment of the present disclosure. On a general level, the steam sterilizer system comprises a container 2 for water, a steam generator 4 and a sterilizer chamber 6. Fresh or recycled liquid water may be supplied to the container 2. Water from the container 2 can be delivered to the steam generator 4. The steam generator 4 comprises a heat exchanger 8 configured to receive a heat transfer medium for transferring heat to the liquid water in the steam generator 4, whereby the steam generator 4 turns the liquid water into steam. The steam is then passed from the steam generator 4 to the sterilizer chamber 6. Inside the sterilizer chamber 6 articles, such as medical goods, to be sterilized can be subjected to steam generated by the steam generator 4.

The received articles in the sterilizer chamber 6 are sterilized in a sterilization process. Such a sterilization process may include a number of different steam treatment stages. During such steam treatment stages, steam may repeatedly be delivered from the steam generator 4 to the sterilizer chamber 6. As previously explained in this disclosure, traditionally after completion of a steam treatment stage, the residual steam has been drained to the sewage system 10. This is not only a waste of thermal energy (considering the heat of the steam) but also a waste of water, which is required to cool down the residual steam before it can be drained to the sewage system. Therefore, in accordance with the general inventive concept, the steam sterilizer system has been provided with a steam recycling arrangement 12.

The steam recycling arrangement 12 is configured to receive at least a part of the residual steam that is present in the sterilizer chamber 6 after a steam treatment stage of the sterilization process. As has been previously explained, recovery of residual steam may be particularly advantageous after late steam treatment stages, but may also be conceivable after completion of earlier steam treatment stages. The steam recycling arrangement 12 is configured to pass the residual steam, from the sterilizer chamber 6, as a heat transfer medium through the heat exchanger 8 so as to transfer heat from the residual steam to the liquid water in the steam generator 4.

The steam recycling arrangement 12 comprises a compressor 14 which is configured to receive the residual steam from the sterilizer chamber 6 and compress the received residual steam to a higher pressure before the steam recycling arrangement 12 passes the compressed residual steam to the heat exchanger 8.

The steam recycling arrangement 12 may also comprise an intermediate steam storage 16, located between the compressor 14 and the steam generator 4. Thus, the compressor 14 may be located in a fluid path between the sterilizer chamber 6 and the intermediate steam storage 16 for compressing the residual steam form the sterilizer chamber 6 before it enters the intermediate steam storage 16. The functioning of the intermediate steam storage 16 will be discussed in more detail further down in this disclosure.

The steam sterilizer system 1 may include a control unit 18 which is configured to communicate with various components of the steam sterilizer system 1, and is also configured to control the operation of certain components and to receive input signals from various components. The control unit 18 may be configured to communicate, send/receive instructions, etc. wirelessly or by wire. The control unit 18 may include electronics, processors, memory, electronic instructions, and other elements that are useful to control the system described in this disclosure, including any embodiment thereof. The control unit 18 may control various valves of the steam sterilizer system 1 to control the flow of fluids through different passages, such as the flow of water, steam etc. For instance, a passage 20 from the sterilizer chamber 6 to the compressor 14 may be provided with a valve 22 that is controllable by the control unit 18. The valve 22 may, for instance, be a proportional valve. The control unit 18 may close the valve 22 when residual steam from the sterilizer chamber 6 should not be recovered, and may open the valve 22 when residual steam is to be recovered. Thus, the control unit 18 may be configured to determine that a steam treatment stage of the sterilization process has been completed and, based on such determination, control the valve 22 located upstream of the compressor 14 to allow residual steam to pass from the sterilizer chamber 6 to the compressor 14.

The compressor 14 will increase the pressure of the residual steam and thereby also increase the temperature. There is a possibility that the residual steam may become superheated. It may therefore be desirable to control (e.g. cool) any superheated residual steam since superheated steam provides poor heat transfer in the heat exchanger 8. Turning superheated steam into saturated steam may therefore be desirable. To this end, the steam recycling arrangement 12 may comprise a cooling device 24a, 24b, 24c, 24d. The cooling device may be provided to lower the temperature of superheated residual steam into saturated residual steam. The cooling device may suitably be along a fluid path between the sterilizer chamber 6 and the intermediate steam storage 16. In Fig. 1, there are illustrated three different example locations for spray injecting water onto the superheated residual steam. One of these three different options will normally suffice for achieving saturated residual steam. The three illustrated options are, injection upstream (24a) of the compressor 14, injection directly into (24b) the compressor 14, and injection downstream (24c) of the compressor 14. A further option or supplement, is to use a cooling device in the form of a heat exchanging device 24d, which is here illustrated downstream of the compressor 14. Each one of the exemplified cooling devices 24a-24d, i.e. the spray injectors 24a, 24b, 24c and the heat exchanging device 24d, are shown as being positionable upstream of the intermediate steam storage 16. The steam sterilizer system 1 may include sensing devices 26, such as one or more temperature sensors and one or more pressure sensors. Based on input from such sensing devices 26 the control unit 18 can calculate the saturation temperature, and may accordingly determine a suitable duration for cooling by means of the cooling devices 24a-24d so as to reach the saturation temperature without going into the wet area.

The intermediate steam storage 16 may also function to control superheat. The compressed residual steam may be guided to the intermediate steam storage 16 before the compressed residual steam is passed on to the heat exchanger 8 of the steam generator 4. By having a higher pressure in the intermediate steam storage 16 compared to the pressure in the steam generator 4, an improved heat transfer is achieved via the heat exchanger 8. The intermediate steam storage 16 preferably comprises a pressure vessel. The pressure vessel may be provided with liquid water 28, which may serve dual purposes. One of them being superheat control, the other one being generation of additional steam. As regards the superheat control, the intermediate steam storage 16 may be provided with an inlet 30, which may be at the bottom of the intermediate steam storage 16. The compressed residual steam may be received through that inlet 30, thereby enabling residual steam to rise through the water 28 contained in the intermediate steam storage 16. Thereby, excess heat may be transferred into the water 28. The intermediate steam storage 16 may suitably comprise a screen 32 with a plurality of holes for spreading the transportation of steam bubbles 34 through the water volume 28 in the intermediate steam storage 16. The steam bubbles 34 rise to an upper steam volume 36 above the surface of the water volume 28, and the temperature of the steam becomes lowered as it rises through the water 28. As regards the generation of additional steam, this may be relevant when starting up the system. The intermediate steam storage 16 may therefore be equipped with electric heaters 38 or some other heating arrangement. However, also when the system is up and running, the water 28 may be used for adding new steam in order to compensate for losses. The liquid water 28 in the intermediate steam storage may be delivered from the container 2, for example via an economizer 40 for preheating the supplied water. The control unit 18 may control valves 42, 44 to control water to be delivered from the container 2 to the steam generator 4 and/or the intermediate steam storage 16.

The saturated residual steam may exit the intermediate steam storage 16 through an outlet 46 provided at the top of the intermediate steam storage 16. A passage 48 from the outlet 46 guides the compressed saturated residual steam to the heat exchanger 8 of the steam generator 4. After having passed through the heat exchanger 8, the residual steam will have transferred heat to the water inside the steam generator 4, and will thus have lowered its temperature and can be evacuated from the heat exchanger 8 in the form of condensate at a drain 50. The passage 48 interconnecting the intermediate steam storage 16 with the heat exchanger 8 of the steam generator 4 may suitably be provided with a valve 52 that is controllable by the control unit 18. The valve 52 may thus be closed when it is not time to heat the water in the steam generator 4, and may be opened when it is time to heat the water in the steam generator 4.

It should be understood that although only the heat exchanger 8 for receiving the residual steam is illustrated in the steam generator 4, it is useful to have additional heating elements for turning the water in the steam generator 4 into steam.

As schematically indicated at the passage 48 between the intermediate steam storage 16 and the heat exchanger 8, there may be provided a bypass line 54 (schematically indicated by an arrow) which is selectively controllable (e.g. by means of a valve controllable by the control unit 18) to allow compressed residual steam to bypass the steam generator 4 and be returned to the sterilizer chamber 6. Although the bypass line 54 is illustrated as branching off from the passage 48 between the intermediate steam storage 16 and the heat exchanger 8, any other suitably location upstream of the compressor 14 but downstream of the steam generator 4 may be conceivable. Irrespective of the location of the bypass line, it should be understood that the bypass line does not necessarily have to return the residual steam directly to the sterilizer chamber, but may lead the residual steam to a storage for later use.

The compressor 14 may be used for recovering residual steam from additional sterilizer chambers. Thus, the steam sterilizer system 1 may comprise more than just the sterilizer chamber 6 shown in Fig. 1. For instance, at the branch 56 extending from the passage 20 between the sterilizer chamber 6 and the compressor 14 and by the valve 58 in that branch 56, there may be provided another sterilizer chamber on the other side of that valve 58. Thus, the illustrated sterilizer chamber 6 may be referred to as a first sterilizer chamber, and the other sterilizer chamber on the other side of the valve 58 may be referred to as a second sterilizer chamber. There may, of course, be included more than two sterilizer chambers in a steam sterilizer system 1 of the present disclosure, and steam may similarly be recycled from each.

Similarly, at branch 60 and valve 62 in the passage 48 between the intermediate steam storage 16 and the heat exchanger 8 there may be provided another steam generator on the other side of that valve 62. Such additional steam generator may comprise its own heat exchanger to which residual steam may be guided. The illustrated steam generator 4 may be referred to as a first steam generator, and the other steam generator on the other side of the valve 62 may be referred to as a second steam generator. There may, of course, be included more than two steam generators in a steam sterilizer system 1 of the present disclosure.

The control unit 18 may be configured to control the operation of the different valves and the recovery of residual steam in an effective way. Suitably the timing of the sterilization processes in the different sterilizer chambers may be appropriately adjusted so that when a steam treatment stage in one of the sterilizer chambers has been completed, a steam treatment stage in the other sterilizer chamber is being performed or about to be performed. In this way the control unit 18 may alternatingly control the valves associated with the first sterilizer chamber and the second sterilizer chamber. For example, the control unit 18 may control the valve 22 associated with the first sterilizer chamber 6 to be opened for recovery of residual steam, while the valve 58 associated with the second sterilizer chamber is kept closed, and then at a later point in time the control unit 18 may instead control the valve 58 associated with the second sterilizer chamber to be opened for recovery of residual steam, while the valve 22 associated with the first sterilizer chamber 6 is kept closed. Similarly, the control unit 18 may alternatingly control the valve 52 associated with the first steam generator 4 to be opened while the valve 62 associated with the second steam generator is kept closed, and then vice versa. A further example is for the control unit 18 to control both valves 22 and 58 to be opened in order to simultaneously recover residual steam from the first sterilizer chamber 6 and the second sterilizer chamber.

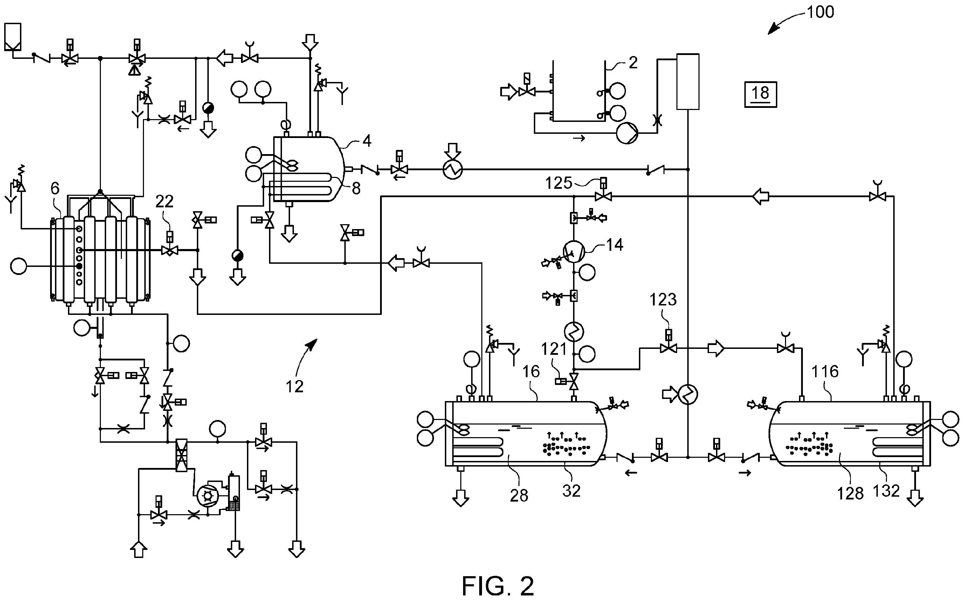

Turning now to Fig. 2, which a schematic view of a steam sterilizer system 100 according to at least another exemplary embodiment of the present disclosure. Many of the parts of the steam sterilizer system 100 in Fig. 2 can be the same as the parts illustrated for the steam sterilizer system 1 in Fig. 1. Thus, similarly to Fig. 1, the steam sterilizer system 100 in Fig. 2 includes a container 2 from which water may be supplied to a steam generator 4 where water is converted into steam and delivered to a sterilizer chamber 6. Residual steam can be received by a recycling arrangement 12. The recycling arrangement 12 comprises a compressor 14 which increases the pressure of the residual steam received from the sterilizer chamber 6 and the compressed residual steam can be passed to and through a heat exchanger 8 of the steam generator 4, wherein the compressed residual steam will function as a heat transfer medium for heating the water in the steam generator 4.

The steam sterilizer system 100 in Fig. 2 comprises a intermediate steam storage 16. Similarly to the steam sterilizer system 1 of Fig. 1, in the steam sterilizer system 100 of Fig. 2, the compressed residual steam may be passed from the compressor 14 via the intermediate steam storage 16 and then to the heat exchanger 8 of the steam generator 4. Although an inlet has been illustrated at the top of the intermediate steam storage 16 (for keeping the drawing figure clear), it should be understood that, in practice, the inlet may suitably be located at the bottom of the intermediate steam storage 16, so as to allow the residual steam to rise through the screen 32 and water 28 in the intermediate steam storage 16, correspondingly to what has already been discussed in relation to Fig. 1.

The intermediate steam storage 16 in Fig. 2 may be referred to as a first intermediate steam storage 16. The steam sterilizer system 100 in Fig. 2 further comprises a second intermediate steam storage 116, to which compressed residual steam may be guided before the compressed residual steam is passed to the heat exchanger 8. The second intermediate steam storage 116 may be structurally similar to the first intermediate steam storage 16, and may for instance comprise a screen 132 and water 128 through which residual steam can rise.

The second intermediate steam storage 116 may for example have a pressure of 150-300 kPa. The second intermediate steam storage 116 can be used for compression of steam held in saturation balance with water that in turn can be used for pre-heating of feed water in economizers, or for a second compression stage from the lower steam pressure 150-300 kPa in the second intermediate steam storage 116 to the first intermediate steam storage 16 (which may have a higher steam pressure of e.g. 500-900 kPa). Having two intermediate steam storages 16, 116 may conserve more energy as the lowest possible pressure for evacuation of the sterilizer chamber 6 can be much lower since the compression ratio also becomes lower. The same compressor 14 can be used for different operating modes and flow may be directed with different valves and piping between the different vessels depending on priority and availability.

The control unit 18 may be configured to operate the steam recycling arrangement in a first storage operating mode by controlling the compressed residual steam to be initially provided to the first intermediate steam storage 16 until the pressure inside the sterilizer chamber 6 has dropped below a predefined pressure level. Thus, the first storage operating mode for steam conservation from the higher pressure may be a controlled flow from the sterilizer chamber 6 through valve 22, via the compressor 14, and via open valve 121 to the first intermediate steam storage 16. In this first storage operating mode, valves 123 and 125) are kept closed. When the pressure in the sterilizer chamber 6 drops, the compression ratio will after a while become too large to effectively continue in the first storage operating mode. The control unit 18 may then operate the steam recycling arrangement 12 in a second storage operating mode when the pressure inside the sterilizer chamber 6 has dropped below the predefined pressure level. The control unit 18 will control the compressed residual steam to be provided to the second intermediate steam storage 116 instead of to the first intermediate steam storage 16. Thus, the second storage operating mode is then instead targeting the second intermediate steam storage 116 with its lower counter pressure. In the second storage operating mode the valve 121 is closed and valve 123 is opened. Valve 125 is still closed. The control unit 18 is also configured to operate the steam recycling arrangement 12 in a third operating mode, which may be referred to as an inter-storage operating mode. In the inter-storage operating mode, the control unit 18 controls the compressed residual steam stored in the second intermediate steam storage 116 to be forwarded to the first intermediate steam storage 16 via a compressor 14 to further increase the pressure of the residual steam exiting the second intermediate steam storage 116. This may be the same compressor 14 located between the sterilizer chamber and the first intermediate steam storage 16, or a different compressor. This is enabled when both valves 22 and 123 are closed, while valves 121 and 125 are open. The inter-storage operating mode will increase pressure and energy in the first intermediate steam storage 16 for use in the steam generator 4. It should be understood that direct use of energy from the second intermediate steam storage 116 for heating is also possible.

Similarly to the steam sterilizer system 1 of Fig. 1, the steam sterilizer system 100 of Fig. 2 may include one or more additional sterilizer chambers and one or more additional steam generators.

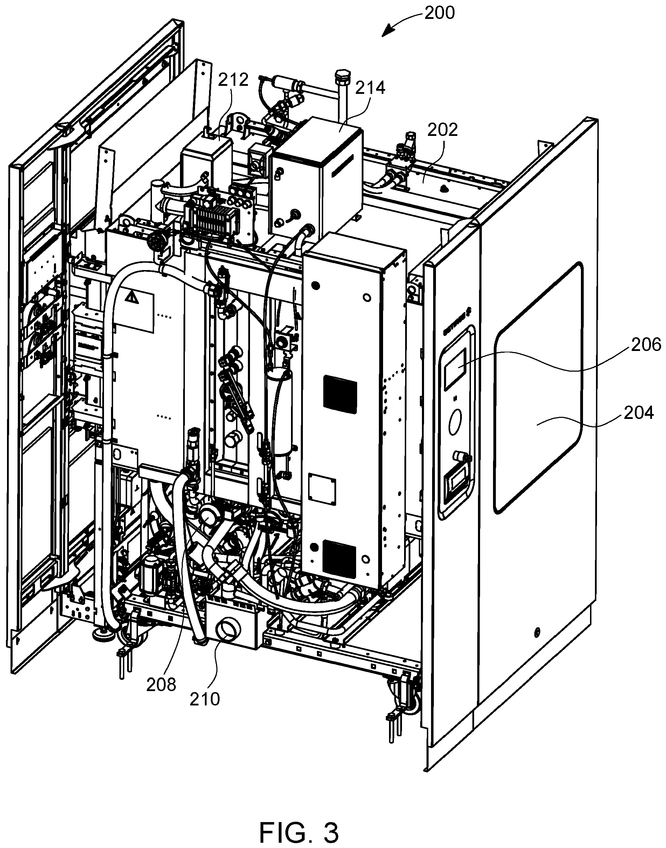

Fig. 3 illustrates an example of a steam sterilizer 200 which could be used with the system of the present disclosure including any exemplary embodiment thereof, such as with either one of the sterilizer systems 1 and 100 in Figs. 1 and 2, respectively. The steam sterilizer 200 includes a sterilizer chamber 202, which may for instance correspond to the sterilizer chamber 6 in Figs. 1 and 2. The sterilizer chamber 202 is closed by a door 204 during the sterilization process, and the door 204 may be opened to load non-sterile articles into the sterilizer chamber 202 or unload sterilized articles from the sterilizer chamber 202. In some exemplary embodiments, the steam sterilizer 200 may have another door on the opposite side of the sterilizer chamber 202, wherein loading is performed on one side and unloading is performed on the other side of the sterilizer chamber 202. The steam sterilizer 200 may have an operating panel 206 enabling the personnel to select a suitable sterilization process for the articles to be sterilized. The steam sterilizer 200 may comprise a steam generator 208, such as the steam generator 4 in Figs. 1 and 2. Accordingly, the steam sterilizer 200 in Fig. 3 may include a steam generator 4 and sterilizer chamber 6 similar to those illustrated in Figs. 1 and 2. At the bottom of the steam sterilizer 200 there is provided a drain 210 for evacuating condensed steam and/or other fluids from the steam sterilizer 200. This drain 210 is typically different and separate from the port (shown in Figs. 1 and 2) for steam leaving the sterilizer chamber 6 through valve 22 towards the compressor 14, which is not visible in this view. At the top of the steam sterilizer 200 there may be installed a feed water tanks 212, and also a header tank 214 for cooling water.

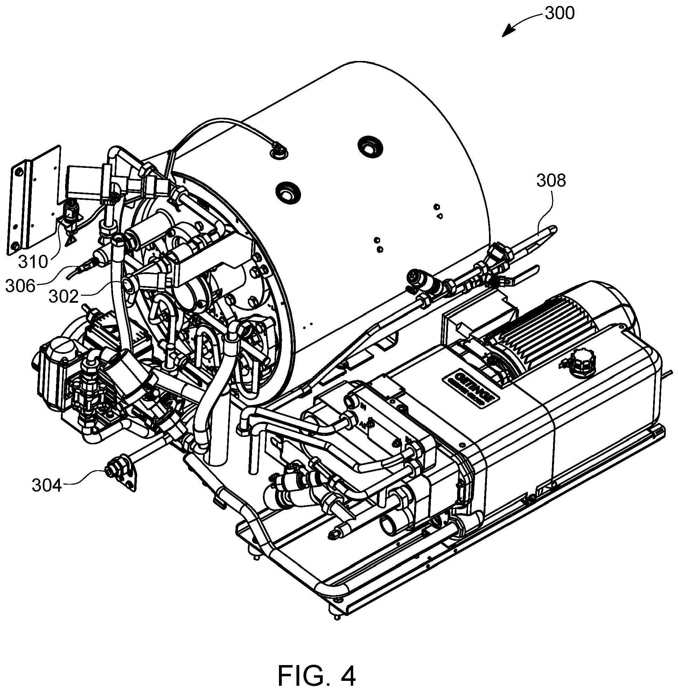

Fig. 4 illustrates an example of a steam generator 300 which could be used with the system of the present disclosure including any exemplary embodiment thereof, such as with either one of the sterilizer systems 1 and 100 in Figs. 1 and 2, respectively. Thus, the steam generator 300 may, for instance, represent a detailed example of the steam generator 4 in Figs. 1 and 2. Furthermore, the steam generator 300 of Fig. 4 may be integrated as a steam generator 208 in the steam sterilizer 200 of Fig. 3. The steam generator 300 shown in Fig. 4 includes an inlet 302 to its heat exchanger. Thus, the compressed residual steam will be received through this inlet 302. The steam generator 300 also includes an outlet 304 from the heat exchanger, through which residual condensate may be evacuated. A sensor 306 may be provided for detecting the water level in the steam generator, whereby a control unit (such as previously discussed control unit 18) may determine when it is time to supply the steam generator 300 with more water through feed water piping 308. The steam generator 300 may also comprise a pressure transmitter 310 for power/pressure control.

This disclosure includes steam sterilizer systems, steam recycling arrangements, and methods of operating both, in particular for sterilizing articles for medical use. In particular, this disclosure includes steam sterilizer systems for efficiently recovering thermal energy of residual steam the for use in subsequent steam treatment stages. It should be understood that various features disclosed herein are contemplated and disclosed in their various combinations and sub-combinations.

## Claims

1. A steam sterilizer system, comprising:
- a sterilizer chamber configured to receive articles, such as medical goods, to be sterilized in a sterilization process in the sterilizer chamber,
- a steam generator configured to receive liquid water and turn the liquid water into steam to be supplied to the sterilizer chamber, the steam generator comprising a heat exchanger, and
- a steam recycling arrangement configured to receive at least a part of the residual steam that is present in the sterilizer chamber after a steam treatment stage of the sterilization process, wherein the steam recycling arrangement is configured to pass the residual steam from the sterilizer chamber, as a heat transfer medium, through said heat exchanger so as to transfer heat from the residual steam to the liquid water in the steam generator,
wherein the steam recycling arrangement comprises a compressor configured to receive the residual steam from the sterilizer chamber and compress the received residual steam to a higher pressure before the steam recycling arrangement passes the compressed residual steam to the heat exchanger.

2. The steam sterilizer system according to claim 1, wherein the steam recycling arrangement comprises an intermediate steam storage to which the compressed residual steam is guided before the compressed residual steam is passed on to the heat exchanger, the pressure in the intermediate steam storage being higher than the pressure in the steam generator, in particular wherein the intermediate steam storage comprises a pressure vessel.

3. The steam sterilizer system according to claim 2, wherein the intermediate steam storage has an inlet at the bottom of the intermediate steam storage through which compressed residual steam is receivable, thereby enabling residual steam to rise through water contained in the intermediate steam storage.

4. The steam sterilizer system according to any one of claims 2-3, wherein the intermediate steam storage comprises a screen with a plurality of holes for spreading the transportation of steam bubbles through a water volume in the intermediate steam storage, wherein the steam bubbles rise to an upper steam volume above the surface of the water volume.

5. The steam sterilizer system according to any one of claims 2-4, wherein the compressor is located in a fluid path between the sterilizer chamber and the intermediate steam storage for compressing the residual steam from the sterilizer chamber before it enters the intermediate steam storage.

6. The steam sterilizer system according to any one of claims 1-5, wherein the steam recycling arrangement comprises a cooling device configured to lower the temperature of superheated residual steam into saturated residual steam, in particular wherein the cooling device is along a fluid path between the sterilizer chamber and an intermediate steam storage.

7. The steam sterilizer system according to claim 6, wherein the cooling device is configured to spray water, in particular to spray water into the compressor.

8. The steam sterilizer system according to claim 6, wherein the cooling device is in the form of a heat exchanging device provided between the compressor and the intermediate steam storage, or in the form of an injector configured to spray water onto superheated residual steam upstream or downstream of the compressor.

9. The steam sterilizer system according to any one of claims 1-8, further comprising a control unit configured to determine that a steam treatment stage of the sterilization process has been completed and, based on such determination, control a valve located upstream of the compressor to allow residual steam to pass from the sterilizer chamber to the compressor.

10. The steam sterilizer system according to any one of claims 1-9, wherein the intermediate steam storage is a first intermediate steam storage, wherein the steam recycling arrangement further comprises a second intermediate steam storage to which the compressed residual steam is guidable before the compressed residual steam is passed to the heat exchanger.

11. The steam sterilizer system according to claim 10 when dependent on claim 9, wherein the control unit is configured to operate the steam recycling arrangement in a first storage operating mode by controlling the compressed residual steam to be initially provided to the first intermediate steam storage until the pressure inside the sterilizer chamber has dropped below a predefined pressure level.

12. The steam sterilizer system according to any one of claim 10-11 when dependent on claim 9, wherein the control unit is configured to operate the steam recycling arrangement in a second storage operating mode when the pressure inside the sterilizer chamber has dropped below said predefined pressure level, by controlling the compressed residual steam to be provided to the second intermediate steam storage instead of to the first intermediate steam storage.

13. The steam sterilizer system according to any one of claims 10-12 when dependent on claim 9, wherein the control unit is configured to operate the steam recycling arrangement in an inter-storage operating mode by controlling the compressed residual steam stored in the second intermediate steam storage to be forwarded to the first intermediate steam storage via the compressor to further increase the pressure of the residual steam exiting the second intermediate steam storage.

14. The steam sterilizer system according to any one of claims 12-13, wherein the steam recycling arrangement comprises a plurality of controllable valves for controlling the flow of steam to and from the first and second intermediate steam storages, wherein the control unit is configured to control the controllable valves to switch between said different operating modes.

15. The steam sterilizer system according to any one of claims 1-14, wherein the steam recycling arrangement further comprises a bypass line extending from a location downstream of the compressor but upstream of the steam generator, wherein the bypass line is selectively controllable to allow compressed residual steam to bypass the steam generator and be returned to the sterilizer chamber.

16. The steam sterilizer system according to any one of claims 1-15, wherein the sterilizer chamber is a first sterilizer chamber and the steam generator is a first steam generator, wherein the steam sterilizer system further comprises:
- a second sterilizer chamber configured to receive articles, such as medical goods, to be sterilized,
- a second steam generator configured to receive liquid water and turn the liquid water into steam to be supplied to the second sterilizer chamber, the second steam generator comprising a heat exchanger,
- wherein the steam recycling arrangement is configured to also guide the residual steam as a heat transfer medium through the heat exchanger of the second steam generator so as to transfer heat from the residual steam to the liquid water in the second steam generator.
